# EUROPEAN PATENT APPLICATION

(11) **EP 4 756 699 A1**
(43) Date of publication of application: **10.06.2026**
(21) Application number: 24856515.2
(22) Date of filing: 22.08.2024
(51) Int. Cl.: G06Q 50/18, G06F 16/90, G06F 40/44, G06F 40/56, G06N 3/08, G06N 3/045, G06N 3/0475, G06N 5/022, G06N 20/00, G06Q 10/00, G06Q 10/10, G06Q 10/0637, G06Q 40/04, G06Q 40/06, G06Q 50/10, G06Q 50/26, G16H 20/00, G16H 50/20, G16H 80/00

(54) **INFORMATION PROVISION DEVICE, INFORMATION PROVISION METHOD, AND INFORMATION PROVISION PROGRAM**

(30) Priority: 24.08.2023 JP 2023136096; 24.08.2023 JP 2023136197; 31.08.2023 JP 2023141614; 01.09.2023 JP 2023142049; 01.09.2023 JP 2023142104; 01.09.2023 JP 2023142257; 05.09.2023 JP 2023143536; 11.09.2023 JP 2023146821; 11.09.2023 JP 2023147153; 14.09.2023 JP 2023149192; 14.09.2023 JP 2023149239; 14.09.2023 JP 2023149567; 14.09.2023 JP 2023149590; 14.09.2023 JP 2023149611; 14.09.2023 JP 2023149624
(71) Applicant: SoftBank Group Corp., Tokyo 105-7537 (JP)
(72) Inventor: SON, Masayoshi, Tokyo 105-7537 (JP)
(74) Representative: SSM Sandmair
(86) International application number: PCT/JP2024/029820
(87) International publication number: WO 2025/041827

(57) **Abstract**

An information providing apparatus (10) includes a debate unit (33) and a judging unit (34). The debate unit (33) performs a debate regarding a legal case by a plurality of lawyer accounts that make respectively different arguments, the lawyer accounts being set in a language model that is generated by learning data stored in a database (20a) regarding legal cases and that generates a response to an input prompt. The judging unit (34) judges superiority or inferiority of the arguments based on a result of the debate by the debate unit (33), by a judging account set in the language model.

## Description

### Technical Field

The disclosed embodiments relate to an information providing apparatus, an information providing method, and an information providing program.

### Background Art

Conventionally, a system is known that generates, by using a language model, a response sentence to a question sentence input by a user (see, for example, Patent Literature 1).

### Citation List

### Patent Literature

[Patent Literature 1] JP 2022-503838 A

### Summary of Invention

### Technical Problem

In the conventional technology, while response sentences are generated based on news articles, utilization of a language model specialized in a specific field has not been considered.

The present invention has been made in view of the above, and an object thereof is to effectively utilize a language model specialized in a specific field.

### Solution to Problem

An information providing apparatus according to an aspect of the embodiments includes: a debate unit that performs a debate regarding a legal case by a plurality of lawyer accounts that make respectively different arguments, the lawyer accounts being set in a language model that is generated by learning data stored in a database regarding legal cases and that generates a response to an input prompt; and a judging unit that judges superiority or inferiority of the arguments based on a result of the debate by the debate unit, by a judging account set in the language model.

### Effects of Invention

According to an aspect of embodiments, it is possible to effectively utilize a language model specialized in a specific field.

### Brief Description of the Drawings

FIG. 1 is a diagram explaining an outline of an information providing apparatus according to a first embodiment.
FIG. 2 is a functional block diagram illustrating a configuration example of the information providing apparatus according to the first embodiment.
FIG. 3 is a flowchart illustrating a processing procedure executed by the information providing apparatus according to the embodiment.
FIG. 4 is a flowchart illustrating a processing procedure executed by the information providing apparatus according to the embodiment.
FIG. 5 is a diagram schematically illustrating an example of a computer hardware configuration functioning as the information providing apparatus.
FIG. 6 is a diagram explaining a flow of processing of an information providing apparatus according to a second embodiment.
FIG. 7 is a diagram illustrating a configuration of an information providing system including an information providing apparatus according to a third embodiment.
FIG. 8 is a diagram explaining a debate.
FIG. 9 is a diagram explaining an outline of an information providing apparatus according to a fourth embodiment.
FIG. 10 is a diagram explaining an outline of an information providing apparatus according to a fifth embodiment.
FIG. 11 is a diagram explaining a debate.
FIG. 12 is a flowchart illustrating a processing procedure of a debate.
FIG. 13 is a diagram explaining an outline of an information providing apparatus according to a sixth embodiment.
FIG. 14 is a diagram illustrating an outline of a debate according to the sixth embodiment.
FIG. 15 is a diagram illustrating an example of a prompt according to the sixth embodiment.
FIG. 16 is a flowchart illustrating a processing procedure executed by the information providing apparatus according to the sixth embodiment.

### Detailed Description of Embodiments

Hereinafter, the present invention will be described through embodiments, but the following embodiments do not limit the invention according to the claims. In addition, not all combinations of features described in the embodiments are essential to the solution of the invention.

### (1. First Embodiment)

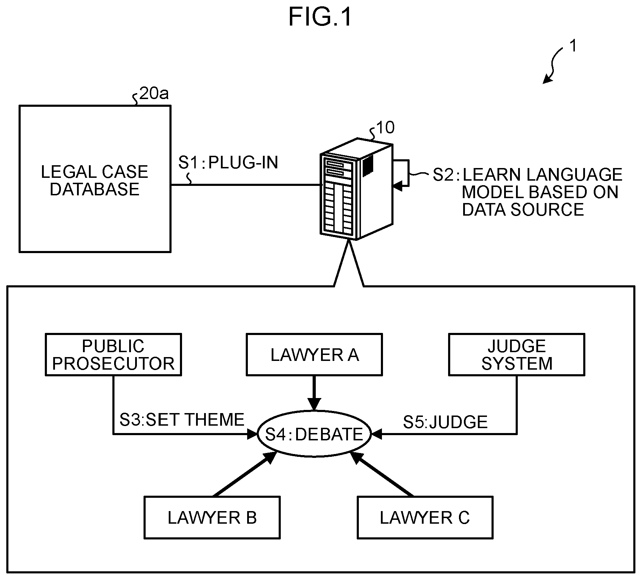

A flow of processing of an information providing apparatus according to a first embodiment will be described with reference to FIG. 1. FIG. 1 is a diagram explaining an outline of the information providing apparatus according to the first embodiment. FIG. 1 illustrates a configuration of an information providing system 1 including an information providing apparatus 10 according to the first embodiment.

As illustrated in FIG. 1, the information providing system 1 includes the information providing apparatus 10 and a legal case database 20a. The legal case database 20a is a database that stores data regarding various laws. For example, the legal case database 20a stores data regarding various laws concerning civil cases and criminal cases, and past judicial precedents. Note that the legal case database 20a corresponds to an example of a database according to the embodiment.

The information providing apparatus 10 is an apparatus to which each device including the legal case database 20a is connected via a plug-in. Each device includes every device such as various servers, vehicles, and smartphones. In the information providing system 1, the information providing apparatus 10 realizes the highest peak of artificial intelligence by being connected to each device via a plug-in.

For example, in the information providing system 1, semiconductor chips manufactured by the same manufacturer are incorporated into the information providing apparatus 10 and each device including the legal case database 20a. Each of these semiconductor chips may itself perform machine learning or deep learning.

In addition, as the semiconductor chips used in the information providing apparatus 10 and the legal case database 20a, chips of sizes suitable for respective housings are used. For example, assuming that the chip size of the information providing apparatus 10 is XL size, L size is used for servers and vehicles, and S or M size is used for user terminals. Further, for a terminal having a housing size smaller than that of the user terminal, SS size is used, and it is put into a SoC (System on a chip) to be made into one chip. Note that, regarding the XL size, the number of semiconductor chips is 200 tiles, the L size is 50 tiles, the M size is 20 tiles, the S size is 10 tiles, and the SS size is 2 tiles, but the number of tiles of each size is an example and is not limited to the above. If one wants to attach accessories such as a camera and a microphone to the user terminal, a chip dedicated to the accessories, which is different from the semiconductor chip, may be set in a vacant space near the semiconductor chip in the SoC.

In this way, in the information providing system 1, by using semiconductor chips of the same manufacturer for various devices connected to the information providing apparatus 10, the information providing system 1 can be kept in a secure state. That is, in the information providing system 1, hacking, virus infection, and deep fakes can be avoided with high accuracy, and privacy between data source providers including the information providing apparatus 10 and the legal case database 20a can be ensured.

In the present embodiment, the information providing apparatus 10 performs learning of a model based on a data source acquired from the legal case database 20a. The model is a language model. As the language model, for example, ChatGPT of OpenAI is known (Reference: https://openai.com/blog/chatgpt). The language model may be a language model such as GAN (Generative Adversarial Networks) or VAE (Variational Autoencoder) using a neural network.

In the present embodiment, the language model generates a text of a response to a text of an inquiry (hereinafter, prompt) input by a user. In addition, the language model is learned in advance using data sources. The learning of the language model may be performed by a known machine learning method.

Thereby, the language model becomes able to generate a response based on the data source. In the present embodiment, the information providing apparatus 10 performs a plug-in connection with the legal case database 20a (Step S1), and performs learning of the language model based on data stored in the legal case database 20a (Step S2).

In this way, the information providing apparatus 10 causes the language model to learn various data stored in the legal case database 20a. Thereby, the language model generates a response to an input prompt based on the learned legal cases. That is, the information providing apparatus 10 can generate a language model specialized in the legal field.

In the present embodiment, a virtual law office is designed using the language model specialized in the legal field. Specifically, in the present embodiment, accounts (roles) of "Lawyers A to C", "Public Prosecutor", and "Judge System" are set in advance by a system prompt. "Lawyers A to C" are accounts that make mutually different arguments. "Public Prosecutor" is an account that determines a theme of a debate by Lawyers A to C. Note that "Public Prosecutor" may play a role as a plaintiff and also play a role of making an argument on the plaintiff side. "Judge System" is an account that judges superiority or inferiority of the arguments of Lawyers A to C.

In the present embodiment, an argument by each of the Lawyer A to C accounts is input to the language model as a prompt, and it is set as a system prompt so that other lawyer accounts debate against the input prompt.

By such setting, it becomes possible for "Lawyers A to C" to voluntarily perform a debate using the language model. For example, as illustrated in FIG. 1, a theme of the debate is set by "Public Prosecutor" (Step S3). The theme here corresponds to contents of consultation with a corporate lawyer. Note that the theme may relate to a litigation case or the like currently held by a company, or may be a fictitious one.

"Lawyers A to C" perform a debate along the theme set by "Public Prosecutor" (Step S4). For example, "Lawyers A to C" make respectively different arguments and perform a debate on logic supporting the arguments. For example, "Lawyers A to C" debate with each other by developing logic supporting their own arguments from related laws and past judicial precedents.

Thereafter, "Judge System" performs a judge based on contents of the debate (Step S5). For example, "Judge System" judges which judge system's argument is superior when a predetermined termination condition is satisfied, such as when the debate boils down. For example, "Judge System" collates related laws and past judicial precedents, and performs a judge on superiority or inferiority of the argument by each lawyer.

Then, "Judge System" performs a judge on the argument and logic of each lawyer from past judicial precedents and the like, and selects the most excellent argument. In this way, the information providing apparatus 10 performs learning of the language model based on data acquired from the legal case database 20a.

Then, the information providing apparatus 10 causes each account set in advance to debate using the language model, and derives a conclusion of the debate from the debate contents. For example, the information providing apparatus 10 can perform such a debate many times as compared with a debate by actual lawyers. For example, it is possible to perform a debate on the same theme many times, and cause an argument of a winner of a first debate and an argument of a winner of a second debate to debate with each other.

That is, the information providing apparatus 10 can brush up each argument by repeating such debates. Thereby, the information providing apparatus 10 can make a significant argument as compared with an actual lawyer.

Therefore, according to the information providing apparatus 10 according to the embodiment, it is possible to effectively utilize a language model specialized in a specific field.

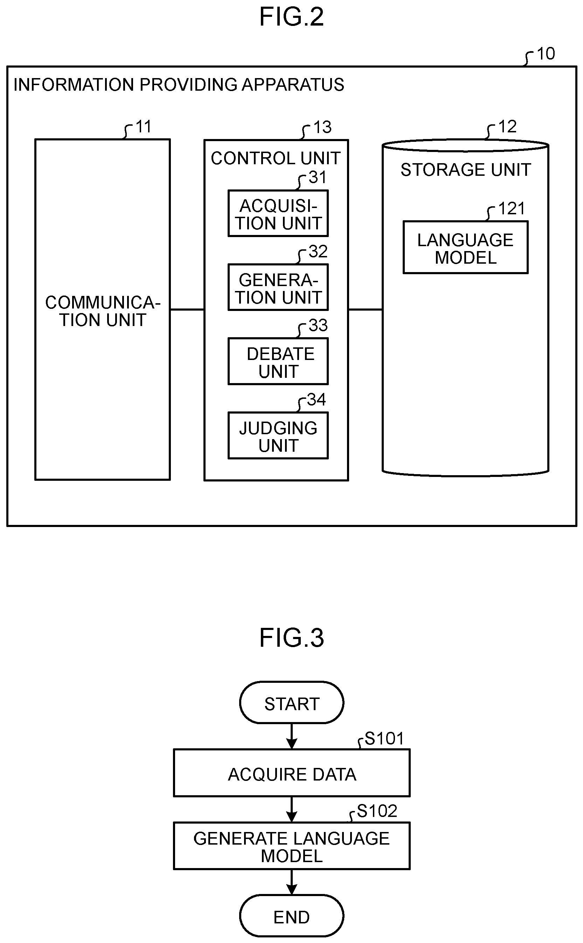

A configuration of the information providing apparatus 10 will be described with reference to FIG. 2. FIG. 2 is a functional block diagram illustrating a configuration example of the information providing apparatus according to the first embodiment.

As illustrated in FIG. 2, the information providing apparatus 10 includes a communication unit 11, a storage unit 12, and a control unit 13.

The communication unit 11 performs transmission and reception of information to and from the legal case database 20a and the like via a network.

The storage unit 12 is realized by, for example, a semiconductor memory element such as a RAM (Random Access Memory) or a flash memory (Flash Memory), or a storage device such as an HDD (Hard Disk Drive), an SSD (Solid State Drive), or an optical disk. The storage unit 12 stores various programs, various data, and the like. The storage unit 12 stores language model information 121.

The language model information 121 is information regarding a language model. For example, the language model information 121 is information regarding parameters for constructing a language model. Such parameters are, for example, weights and biases of a neural network.

The control unit 13 is a controller, and includes, for example, a microcomputer having a CPU (Central Processing Unit), a ROM (Read Only Memory), a RAM, an input/output port, and the like, and various circuits. In addition, the control unit 13 may be configured by hardware such as an integrated circuit such as an ASIC (Application Specific Integrated Circuit) or an FPGA (Field Programmable Gate Array). The control unit 13 has an acquisition unit 31, a generation unit 32, a debate unit 33, and a judging unit 34.

The acquisition unit 31 acquires data from a data source provider. For example, the acquisition unit 31 acquires data regarding legal cases from the legal case database 20a (see FIG. 1). The acquisition unit 31 acquires data, for example, every time the legal case database 20a is updated.

The generation unit 32 generates a language model. Specifically, the generation unit 32 learns a language model based on data sources regarding legal cases. In addition, the generation unit 32 updates parameters of a model by learning. The generation unit 32 may regenerate a model at each timing of learning, or may reflect a data source of a difference from previous learning on the model.

The debate unit 33 performs a debate by a plurality of lawyer accounts whose arguments are different from each other, using a language model that is generated by learning data stored in a database regarding legal cases and that generates a response to an input prompt.

First, the debate unit 33 generates a plurality of lawyer accounts, a public prosecutor account, and a judge system account by a system prompt of a language model. For example, each lawyer account is set to make a different argument, and is further set to perform a debate supporting its own argument.

Regarding the public prosecutor account, it is set to set the setting of the theme of the debate. Note that a public prosecutor account may be set to perform a debate with each lawyer account as a plaintiff. In addition, regarding the judge system account, it is set to judge a debate result by each lawyer account or a debate result by each lawyer account and a public prosecutor account.

The debate unit 33 sets these accounts and then sets a theme of the debate. For example, as the theme of the debate, one arbitrarily set by a user may be used. For example, in this case, data when the user makes a request to a lawyer may be input to a language model as a theme.

In addition, the debate unit 33 may use contents of consultation set by a user as a theme of a debate. For example, contents of consultation here include consultation on whether or not a client company should file a lawsuit, and consultation on contract contents of a contract document that is advantageous to the client company.

The judging unit 34 uses a language model to judge an argument based on a debate result by the debate unit 33. The judging unit 34 corresponds to a judge system account. The judging unit 34 judges argument of each lawyer account at a stage where a debate by the lawyer accounts has boiled down.

For example, the judging unit 34 inputs each argument by each lawyer account to a language model as a prompt, and judges which argument is superior among these arguments.

More specifically, the judging unit 34 judges the most excellent argument by collating with related laws and past judicial precedents by a language model. In addition, when a theme of a debate is whether or not to file a lawsuit, the judging unit 34 determines a conclusion thereof from debate result by each lawyer account.

In addition, when a theme of a debate is contract contents of a contract document, the judging unit 34 determines a draft of a contract document based on the debate result by each lawyer account. Then, a result by the judging unit 34 is output to a user terminal of a user (not illustrated).

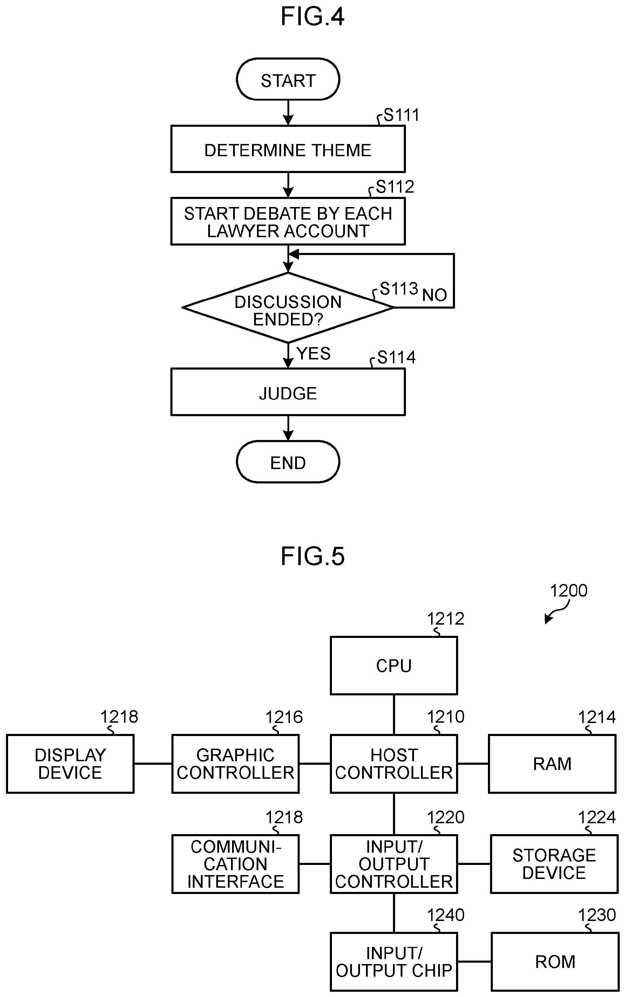

Next, a processing procedure executed by the information providing apparatus 10 according to the embodiment will be described with reference to FIGS. 3 and 4. FIGS. 3 and 4 are flowcharts illustrating the processing procedure executed by the information providing apparatus 10 according to the embodiment.

First, a generation procedure of a language model will be described with reference to FIG. 3. As illustrated in FIG. 3, the information providing apparatus 10 first acquires data regarding legal cases from the legal case database 20a (Step S101). Subsequently, the information providing apparatus 10 generates a language model based on data acquired from the legal case database 20a (Step S102), and ends the process.

Next, a debate process using a language model will be described with reference to FIG. 4. As illustrated in FIG. 4, the information providing apparatus 10 first determines a theme of a debate (Step S111). Subsequently, the information providing apparatus 10 starts a debate by each lawyer account (Step S112).

Subsequently, the information providing apparatus 10 determines whether or not discussion by each lawyer account has ended (Step S113), and when determining that the discussion has not ended (Step S113; No), repeatedly executes the process of Step S113 until the discussion ends.

In addition, when determining that the discussion by each lawyer account has ended (Step S113; Yes), the information providing apparatus 10 judges each argument based on a debate result (Step S114), and ends the process.

According to the present embodiment, by setting a virtual law office using a language model learned based on data stored in the legal case database 20a, it is possible to effectively utilize a language model specialized in a specific field.

FIG. 5 is a diagram schematically illustrating an example of a computer hardware configuration functioning as the information providing apparatus. A program installed in a computer 1200 can cause the computer 1200 to function as one or a plurality of "units" of the apparatus according to the present embodiment, or cause the computer 1200 to execute an operation associated with the apparatus according to the present embodiment or the one or plurality of "units", and/or cause the computer 1200 to execute a process according to the present embodiment or a stage of the process. Such a program may be executed by a CPU 1212 to cause the computer 1200 to execute specific operations associated with some or all of blocks of the flowcharts and block diagrams described in the present specification.

The computer 1200 according to the present embodiment includes the CPU 1212, a RAM 1214, and a graphic controller 1216, which are mutually connected by a host controller 1210. The computer 1200 also includes input/output units such as a communication interface 1222, a storage device 1224, a DVD drive, and an IC card drive, which are connected to the host controller 1210 via an input/output controller 1220. The DVD drive may be a DVD-ROM drive, a DVD-RAM drive, or the like. The storage device 1224 may be a hard disk drive, a solid state drive, or the like. The computer 1200 also includes input/output units such as a ROM 1230 and a keyboard, which are connected to the input/output controller 1220 via an input/output chip 1240.

The CPU 1212 operates according to programs stored in the ROM 1230 and the RAM 1214, thereby controlling each unit. The graphic controller 1216 acquires image data generated by the CPU 1212 in a frame buffer or the like provided in the RAM 1214 or in itself, and causes the image data to be displayed on a display device 1218.

The communication interface 1222 communicates with other electronic devices via a network. The storage device 1224 stores programs and data used by the CPU 1212 in the computer 1200. The DVD drive reads a program or data from a DVD-ROM or the like and provides it to the storage device 1224. The IC card drive reads a program and data from an IC card and/or writes a program and data to an IC card.

The ROM 1230 stores therein a boot program or the like executed by the computer 1200 at the time of activation, and/or a program depending on hardware of the computer 1200. The input/output chip 1240 may also connect various input/output units to the input/output controller 1220 via a USB port, a parallel port, a serial port, a keyboard port, a mouse port, or the like.

The program is provided by a computer-readable storage medium such as a DVD-ROM or an IC card. The program is read from the computer-readable storage medium, installed in the storage device 1224, the RAM 1214, or the ROM 1230 which is also an example of the computer-readable storage medium, and executed by the CPU 1212. Information processing described in these programs is read by the computer 1200 and brings about cooperation between the program and the above-described various types of hardware resources. An apparatus or a method may be configured by realizing an operation or processing of information according to use of the computer 1200.

For example, when communication is executed between the computer 1200 and an external device, the CPU 1212 may execute a communication program loaded in the RAM 1214, and instruct the communication interface 1222 on communication processing based on processing described in the communication program. Under the control of the CPU 1212, the communication interface 1222 reads transmission data stored in a transmission buffer area provided in a recording medium such as the RAM 1214, the storage device 1224, a DVD-ROM, or an IC card, transmits the read transmission data to a network, or writes reception data received from a network to a reception buffer area or the like provided on a recording medium.

In addition, the CPU 1212 may cause all or a necessary part of a file or a database stored in an external recording medium such as the storage device 1224, a DVD drive (DVD-ROM), an IC card, or the like to be read into the RAM 1214, and execute various types of processing on the data on the RAM 1214. The CPU 1212 may then write back the processed data to the external recording medium.

Various types of information such as various types of programs, data, tables, and databases may be stored in a recording medium and subjected to information processing. The CPU 1212 may execute various types of processing on the data read from the RAM 1214, including various types of operations, information processing, conditional judgment, conditional branching, unconditional branching, information retrieval/replacement, and the like, which are described throughout the present disclosure and specified by instruction sequences of the program, and write back a result to the RAM 1214. In addition, the CPU 1212 may search for information in a file, a database, or the like in a recording medium. For example, when a plurality of entries each having an attribute value of a first attribute associated with an attribute value of a second attribute are stored in a recording medium, the CPU 1212 may search for an entry in which the attribute value of the first attribute matches a specified condition from among the plurality of entries, read the attribute value of the second attribute stored in the entry, and thereby acquire the attribute value of the second attribute associated with the first attribute satisfying a predetermined condition.

The program or software module described above may be stored in a computer-readable storage medium on the computer 1200 or in the vicinity of the computer 1200. In addition, a recording medium such as a hard disk or a RAM provided in a server system connected to a dedicated communication network or the Internet can be used as a computer-readable storage medium, thereby providing the program to the computer 1200 via the network.

Blocks in the flowcharts and block diagrams in the present embodiment may represent a stage of a process in which an operation is executed or a "unit" of an apparatus having a role of executing an operation. Specific stages and "units" may be implemented by a dedicated circuit, a programmable circuit supplied with computer-readable instructions stored on a computer-readable storage medium, and/or a processor supplied with computer-readable instructions stored on a computer-readable storage medium. The dedicated circuit may include a digital and/or analog hardware circuit, and may include an integrated circuit (IC) and/or a discrete circuit. The programmable circuit may include a reconfigurable hardware circuit including logical AND, logical OR, exclusive OR, negative logical AND, negative logical OR, and other logical operations, flip-flops, registers, and memory elements, such as a field programmable gate array (FPGA), a programmable logic array (PLA), and the like.

The computer-readable storage medium may include any tangible device capable of storing instructions executed by a suitable device, and as a result, the computer-readable storage medium having instructions stored therein will comprise a product including instructions that can be executed to create means for performing operations specified in the flowcharts or block diagrams. Examples of the computer-readable storage medium may include an electronic storage medium, a magnetic storage medium, an optical storage medium, an electromagnetic storage medium, a semiconductor storage medium, and the like. More specific examples of the computer-readable storage medium may include a floppy (registered trademark) disk, a diskette, a hard disk, a random access memory (RAM), a read only memory (ROM), an erasable programmable read only memory (EPROM or flash memory), an electrically erasable programmable read only memory (EEPROM), a static random access memory (SRAM), a compact disc read only memory (CD-ROM), a digital versatile disc (DVD), a Blu-ray (registered trademark) disc, a memory stick, an integrated circuit card, and the like.

The computer-readable instructions may include either source code or object code written in any combination of one or more programming languages, including assembler instructions, instruction set architecture (ISA) instructions, machine instructions, machine dependent instructions, microcode, firmware instructions, state setting data, or object oriented programming languages such as Smalltalk (registered trademark), JAVA (registered trademark), C++, etc., and conventional procedural programming languages such as the "C" programming language or similar programming languages.

The computer-readable instructions may be provided to a processor of a general purpose computer, a special purpose computer, or other programmable data processing apparatus, or a programmable circuit, locally or via a wide area network (WAN) such as a local area network (LAN), the Internet, etc., to execute the computer-readable instructions so that the processor of the general purpose computer, the special purpose computer, or other programmable data processing apparatus, or the programmable circuit generates means for performing operations specified in the flowcharts or block diagrams. Examples of the processor include a computer processor, a processing unit, a microprocessor, a digital signal processor, a controller, a microcontroller, and the like.

### (2. Second Embodiment)

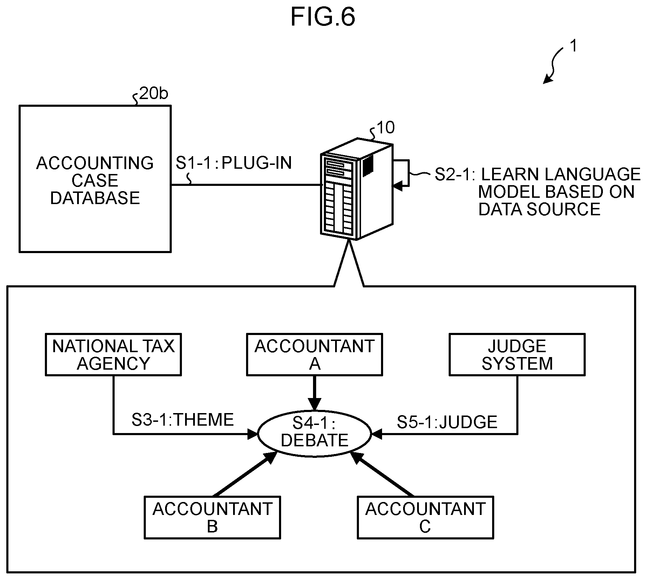

Subsequently, a second embodiment will be described. Note that, in the following, description of parts overlapping with the first embodiment will be omitted. FIG. 6 explains a flow of processing of an information providing apparatus according to the second embodiment. As illustrated in FIG. 6, an information providing system 1 according to the second embodiment includes an information providing apparatus 10 and an accounting case database 20b. The accounting case database 20b is a database that stores data regarding various laws. For example, the accounting case database 20b stores data regarding various laws concerning accounting and past judicial precedents.

The information providing apparatus 10 is an apparatus to which each device including the accounting case database 20b is connected via a plug-in. Each device includes every device such as various servers, vehicles, and smartphones.

In the present embodiment, the information providing apparatus 10 performs learning of a model based on a data source acquired from the accounting case database 20b. The information providing apparatus 10 performs a plug-in connection with the accounting case database 20b (Step S1-1), and performs learning of a language model based on data stored in the accounting case database 20b (Step S2-1).

In this way, the information providing apparatus 10 causes a language model to learn various data stored in the accounting case database 20b. Thereby, the language model generates a response to an input prompt based on learned legal cases. That is, the information providing apparatus 10 can generate a language model specialized in a legal field.

A virtual accounting office is designed using a language model specialized in a legal field. Specifically, accounts (roles) of "Accountants A to C", "National Tax Agency", and "Judge System" are set in advance by a system prompt. "Accountants A to C" are accounts that make mutually different arguments. "National Tax Agency" is an account that participates in a debate by Accountants A to C from a standpoint of the National Tax Agency. Note that "National Tax Agency" may determine a theme of a debate by Accountants A to C as described later. "Judge System" is an account that judges superiority or inferiority of arguments of Accountants A to C.

An argument by each of the Accountant A to C accounts is input to a language model as a prompt, and it is set as a system prompt so that other accountant accounts debate against the input prompt.

By such setting, it becomes possible for "Accountants A to C" to voluntarily perform a debate using a language model. For example, as illustrated in FIG. 6, a theme of a debate is set by "National Tax Agency" (Step S3-1). The theme here corresponds to contents of consultation with a corporate accountant. Note that the theme may relate to an accounting case or the like currently held by a company, or may be a fictitious one.

"Accountants A to C" perform a debate along the theme set by "National Tax Agency" (Step S4-1). For example, "Accountants A to C" make respectively different arguments and perform a debate on logic supporting the arguments. For example, "Accountants A to C" debate with each other by developing logic supporting their own arguments from related laws and past judicial precedents.

Thereafter, "Judge System" performs a judge based on contents of a debate (Step S5-1). For example, "Judge System" judges which accountant's argument is superior when a predetermined termination condition is satisfied, such as when the debate boils down. For example, "Judge System" collates related laws and past judicial precedents, and performs a judge on superiority or inferiority of an argument by each accountant.

Then, "Judge System" performs a judge on the argument and logic of each accountant from past judicial precedents and the like, and selects the most excellent argument. In this way, the information providing apparatus 10 performs learning of a language model based on data acquired from the accounting case database 20b.

Then, the information providing apparatus 10 causes each account set in advance to debate using a language model, and derives a conclusion of the debate from the debate contents. For example, the information providing apparatus 10 can perform such a debate many times as compared with a debate by actual accountants. For example, it is possible to perform a debate on the same theme many times, and cause an argument of a winner of a first debate and an argument of a winner of a second debate to debate with each other.

More specifically, the information providing apparatus 10 performs a debate by a plurality of accountant accounts whose arguments are different from each other, using a language model that is generated by learning data stored in a database regarding accounting cases and that generates a response to an input prompt.

First, the information providing apparatus 10 generates a plurality of accountant accounts, a National Tax Agency account, and a judge system account by a system prompt of a language model. For example, each accountant account is set to make a different argument, and is further set to perform a debate supporting its own argument.

Regarding the National Tax Agency account, it is set to set the setting of a theme of a debate. Note that the National Tax Agency account may be set to participate in discussion of each accountant account from a standpoint of the National Tax Agency. In addition, regarding the judge system account, it is set to judge a debate result by each accountant account or a debate result by each accountant account and the National Tax Agency account.

The information providing apparatus 10 sets these accounts and then sets a theme of a debate. For example, as a theme of the debate, one arbitrarily set by a user may be used. For example, in this case, data when a user makes a request to an accountant may be input to a language model as a theme.

In addition, the information providing apparatus 10 may use contents of consultation set by a user as a theme of a debate.

The information providing apparatus 10 uses a language model to judge an argument based on a debate result. The information providing apparatus 10 judges argument of each accountant account at a stage where the debate by the accountant accounts has boiled down.

For example, the information providing apparatus 10 inputs each argument by each accountant account to a language model as a prompt, and judges which argument is superior among these arguments.

More specifically, the information providing apparatus 10 judges the most excellent argument by collating with related laws and past judicial precedents by a language model. Then, a judge result by the information providing apparatus 10 is output to a user terminal or the like of a user (not illustrated).

That is, the information providing apparatus 10 can brush up each argument by repeating such debates. Thereby, the information providing apparatus 10 can make a significant argument as compared with an actual accountant.

Therefore, according to the information providing apparatus 10 according to the second embodiment, it is possible to effectively utilize a language model specialized in a specific field.

### (3. Third Embodiment)

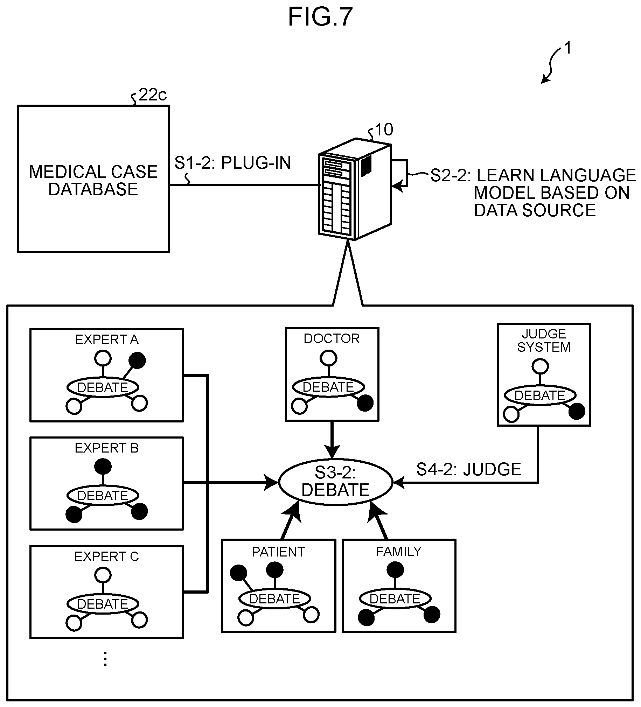

Next, a third embodiment will be described. FIG. 7 is a diagram explaining an outline of an information providing apparatus 10 according to the third embodiment. FIG. 7 illustrates a configuration of an information providing system 1 including the information providing apparatus 10 according to the third embodiment.

As illustrated in FIG. 7, the information providing system 1 includes the information providing apparatus 10 and a medical case database 20c. The medical case database 20c is a database that stores various data regarding medical care. For example, the medical case database 20c stores various data regarding pharmaceuticals, various data regarding insurance, various data regarding medical devices, and the like, including papers and findings regarding medical care and various medical charts. The information providing apparatus 10 performs a plug-in connection with the medical case database 20c (Step S1-2), and performs learning of a language model based on data stored in the medical case database 20c (Step S2-2).

In this way, the information providing apparatus 10 causes a language model to learn various data stored in the medical case database 20c. Thereby, the language model generates a response to an input prompt based on the learned data regarding medical care. That is, the information providing apparatus 10 can generate a language model specialized in a medical field.

In the present embodiment, advice regarding a medical policy for a patient is given using a language model specialized in a medical field. Specifically, in the present embodiment, accounts (roles) of "Doctor", "Patient", "Family", "Experts A to C", and "Judge System" are set in advance by a system prompt of a language model.

Here, it is assumed that accounts in the present embodiment include bot accounts and human accounts. The bot account is a language model for which a system prompt can be set. The bot account outputs a response using a language model. On the other hand, the human account is a human. That is, the human account outputs a response according to an input of a human.

"Doctor", "Experts A to C", "Judge System", "Patient", and "Family" in FIG. 1 are upper accounts. The upper account includes at least one of a bot account or a human account. White circles in FIG. 7 represent bot accounts. Black circles in FIG. 1 represent human accounts.

Hereinafter, a bot account or a human account included in an upper account may be referred to as a lower account. In addition, when it is clear from the preceding and following descriptions and drawings, or when an upper account and a lower account are not distinguished, "upper" and "lower" are omitted and simply referred to as an account.

Hereinafter, description will be given assuming that all accounts are bot accounts. The configuration of FIG. 7 is realized by appropriately replacing a bot account with a human account or adding a human account as a lower account to an upper account.

"Doctor" is an upper account corresponding to, for example, a primary doctor of a patient. FIG. 7 illustrates a case where "Doctor" is one account, but actually, a plurality of accounts are set. Note that the plurality of accounts may be, for example, doctors having different specialized fields (internal medicine, surgery, etc.).

"Patient" is an account corresponding to a patient. "Family" is an account corresponding to a family of the patient. "Experts A to C" are expert accounts involved in treatment of the patient.

Specific examples of the expert account include a medical researcher, a hospital, a nurse, a pharmacist, a pharmaceutical manufacturer, a medical device manufacturer, and the like. "Judge System" is an account that judges a debate result by each account. For example, the judge system judges superiority or inferiority of an argument by each account, and proposes contents thereof to an actual doctor (for example, a primary doctor).

In the present embodiment, an argument by each of a plurality of doctor accounts is input to a language model as a prompt, and it is set as a system prompt so that other doctor accounts debate against the input prompt.

By such setting, it becomes possible for each doctor account to voluntarily perform a debate using a language model. As illustrated in FIG. 7, the information providing apparatus 10 performs a debate regarding a treatment policy for a patient by each account using a language model (Step S3-2).

For example, a debate is started by first inputting various data regarding a patient's age, sex, etc., a patient's medical chart, etc. as a prompt. To explain in more detail, a doctor account is further divided into a plurality of accounts such as Doctor 1, Doctor 2, and Doctor 3, and Doctor 1 is distinguished by a conservative character, Doctor 2 by an aggressive character, and Doctor 3 by a character that emphasizes data, and each performs a debate using the language model. Note that Doctor 1 performs a discussion regarding a medical policy with further subdivided lower accounts such as Doctor 1-1, 1-2, 1-3, and Doctor 2 with Doctor 2-1, Doctor 2-2, Doctor 2-3, and derives a conclusion as Doctor 1 or Doctor 2. Virtual experts engage in billions of debates per second. This enables verification and problem solving from all angles, and allows high-precision diagnosis and proposal of treatment methods based on past medical data to be performed instantly. In addition, Doctor 1 may perform multi-modal data analysis and propose a result of integrating and analyzing various types of medical data such as genetic data, blood data, MRI and X-ray images, Doctor 2 may use a real-time learning model to obtain new data from medical sites around the world in real time and propose that the latest medical information and research results are always incorporated into treatment, and Doctor 3 may have a high-level simulation function and propose to virtually simulate progression of a medical condition, an effect of a treatment method, etc., and predict the most appropriate treatment method and its result in advance. Then, the doctor account may determine a treatment policy using a comprehensive judgment based on respective output results by Doctor 1, Doctor 2, and Doctor 3. As other ideas adopted by the doctor account, it may propose a treatment plan individually optimized based on the background such as genetic information and lifestyle habits of each patient, such as promotion of personalized medicine, or propose to identify a structure of an organ or tissue optimal for a patient by linking with 3D bioprinting and generate an individually customized artificial organ or tissue using 3D bioprinting technology.

For example, a family account derives a conclusion regarding a medical policy as a family by a plurality of accounts set within the family. Each expert account performs a debate with a plurality of accounts for each field regarding the field, and derives a conclusion regarding a medical policy in each field. Then, a doctor account performs a debate regarding a treatment policy for a patient based on debates of these respective fields. Note that a final debate may be performed by all accounts including each account.

For example, a doctor account performs a debate regarding a treatment method, cost, risk, cure rate, and a notification method and notification timing to a patient and family as a treatment policy for the patient. Thereafter, "Judge System" performs a judge based on contents of a debate (Step S4-2). For example, "Judge System" is composed of a plurality of accounts. The plurality of accounts constituting a judge system perform a debate on debate contents of the doctor account and the like, and judge a final medical policy. For example, "Judge System" determines the most appropriate medical policy by collating an argument by each account with data stored in the medical case database 20c when a predetermined termination condition is satisfied, such as when the debate boils down.

Then, "Judge System" proposes a determined medical policy to, for example, the primary doctor of the patient. That is, in the information providing system 1, by using a language model, it is possible to simulate a huge amount of discussion regarding a medical policy by a primary doctor or the like. In addition, since a huge amount of data regarding medical cases is learned in advance in the language model, it is possible to propose a medical policy from various past findings.

In this way, the information providing apparatus 10 according to the embodiment generates a language model based on various findings registered in the medical case database 20c, performs a debate using a language model, and then makes a proposal regarding a medical policy.

Therefore, according to the information providing apparatus 10 according to the embodiment, it is possible to effectively utilize a language model specialized in a specific field.

The information providing apparatus 10 performs a debate using a language model by the following Steps 1 to 5.

Step 1: Set multi-functional virtual experts Virtual experts in different medical fields exist in hundreds of sets, and specific characters are set, for example, "Neurology Expert A", "Neurology Expert B", and "Neurology Expert C" for neurology, and "Cardiology Expert A", "Cardiology Expert B", and "Cardiology Expert C" for cardiology.

### Step 2: Speed up information exchange

These experts perform billions of debates per second while exchanging information based on patient data. Thereby, it is possible to instantly make judgments on the patient's condition, symptoms, treatment methods, and the like.

### Step 3: Handling of data

The virtual experts proceed with the debate while sharing various types of information such as MRI data, genetic data, and blood data. Therefore, an optimal solution from a multilateral perspective is quickly derived.

### Step 4: Self-learning and update

When new knowledge, information, or a treatment method is discovered in a process of a debate, it is immediately reflected in a database and utilized for the debate from the next time. Thereby, diagnosis and treatment can be provided based on the latest medical information.

### Step 5: Formation of consensus

The debate forms a final "consensus" through collision of different opinions and perspectives. Thereby, when diagnosis or treatment proposal to a patient is performed, information and knowledge from various fields are comprehensively incorporated. There may be a plurality of treatment proposal policies.

Although a doctor account has been described above, respective characters are also set for the patient account, a family account, and Expert A to C accounts, and each account is further divided into various characters, and each performs a debate, thereby forming a consensus as a patient, a consensus as a family, and a consensus as Experts A to C.

The information providing apparatus 10 judges a debate result by the information providing apparatus 10 by a judge account set in a language model, and proposes a treatment policy. For example, the information providing apparatus 10 performs a judge when a predetermined termination condition is satisfied, such as when a debate by the information providing apparatus 10 boils down.

For example, the information providing apparatus 10 refers to various data registered in the medical case database 20c, judges a medical policy estimated to be most appropriate among medical policies debated by each doctor account, and proposes a medical policy. Alternatively, a medical policy may be proposed by adding various consensuses such as a consensus as a patient (want to avoid hospitalization as much as possible), a consensus as a family (want to avoid leaving scars as much as possible), and a consensus as Expert A (how much insurance will be paid) to consensus result of a doctor account.

### (Debate when human account is included)

Description has been given so far regarding a debate assuming that the lower account is a bot account. Here, a debate when a human account is included in the lower account will be described. In this case, processing contents of Step 1 and Step 2 described above by the information providing apparatus 10 change.

First, in Step 1, the information providing apparatus 10 changes a configuration so that at least some of the upper accounts include a human account as a lower account. For example, the information providing apparatus 10 replaces a bot account with a human account or newly adds a human account. A user, that is, an existing human is associated with a human account.

Subsequently, in Step 2, the information providing apparatus 10 presents a text output as a response by each account to a user associated with the human account. Then, the information providing apparatus 10 accepts an input of a text from the user. Note that a text of a response output by each account and an operation of outputting a text as a response are referred to as a "statement". The "statement" includes a text input by a user.

The bot account treats a statement of a human account in the same manner as a statement of a bot account. For example, a bot account inputs statements of other bot accounts and a human account to its own language model as a prompt, and further outputs a response.

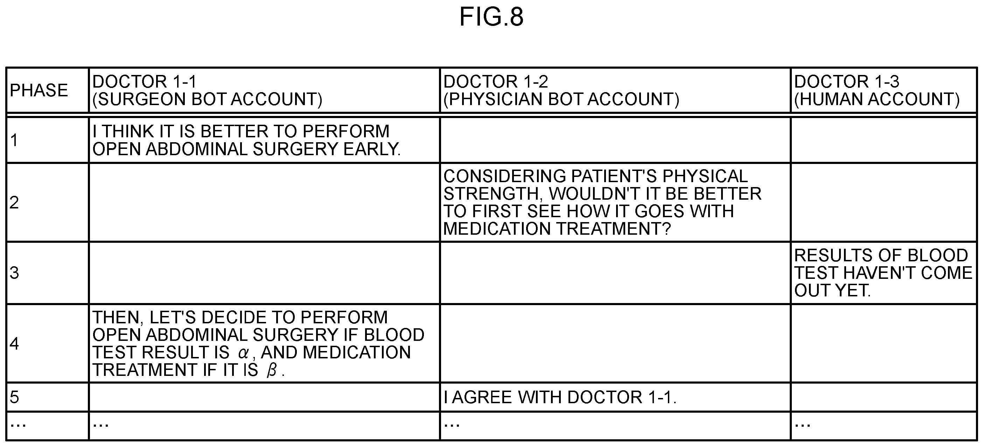

The processing of the information providing apparatus 10 will be described with reference to FIG. 8. FIG. 8 is a diagram explaining a debate. As illustrated in FIG. 8, the information providing apparatus 10 performs a debate according to progress of phases. Here, it is assumed that Doctor 1, which is an upper account, is composed of three lower accounts of Doctor 1-1, Doctor 1-2, and Doctor 1-3.

Doctor 1-1 is a bot account of a surgeon. For example, a language model of Doctor 1-1 outputs a text that prioritizes a surgical treatment method.

Doctor 1-2 is a bot account of a physician. For example, a language model of Doctor 1-2 outputs a text that prioritizes an internal medical treatment method.

Note that the entity of a bot account is a language model, and a debate is realized by the information providing apparatus 10 repeating input of a prompt to the language model. The prompt is, for example, a statement of the language model in the previous phase. What kind of data is emphasized in making a statement may be set by a system prompt. As a result, each bot account has a different tendency of response.

Doctor 1-3 is a human account. A user is associated with Doctor 1-3. In addition, a character (what is emphasized) of Doctor 1-3 may be anything.

As illustrated in FIG. 8, in Phase 1, Doctor 1-1 states, "I think it is better to perform open abdominal surgery early.". Here, the information providing apparatus 10 advances a phase.

In Phase 2, Doctor 1-2 states, "Considering the patient's physical strength, wouldn't it be better to first see how it goes with medication treatment?" based on a result of inputting the statement of Doctor 1-1 in Phase 1 to the language model as a prompt.

Next, in Phase 3, the human account states, "The results of the blood test haven't come out yet.". The statement of the human account is contents input by the user. For example, the information providing apparatus 10 may display a statement of each account on a user's terminal by a chat UI, and accept an input of the user from an input field of the chat UI.

Note that each account including a bot account and a human account does not need to make a statement in all phases. The information providing apparatus 10 only needs to cause at least one account to make a statement in each phase.

In addition, when a human account is included in accounts performing a debate, it is difficult to cause a user to make a statement every time in each of phases that may number in the billions. Therefore, for example, the information providing apparatus 10 may accept a user's statement only in a specific phase, such as once every 10,000 phases.

In addition, the information providing apparatus 10 may accept a user's statement when a condition is satisfied. The condition is a case where a bot account repeats a statement of the same content a certain number of times, or the like. By the bot account using the user's statement as a prompt, it is expected that a stagnant debate starts to proceed toward a conclusion.

In this way, the information providing apparatus 10 presents a response to a user when a response satisfies a condition. Then, the information providing apparatus 10 collects a text input according to the presented response.

In addition, there is a case where a user has knowledge that was not used for learning of a language model of a bot account. In such a case, there is a possibility that a more useful conclusion can be obtained in the debate when a human account is included than in the case of only a bot account.

### (4. Fourth Embodiment)

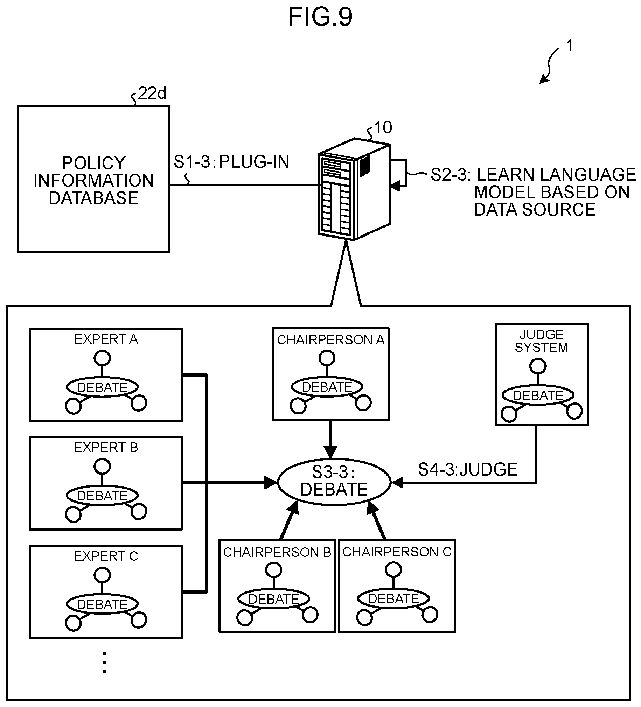

Next, a fourth embodiment will be described. FIG. 9 is a diagram explaining an outline of an information providing apparatus according to the fourth embodiment. FIG. 9 illustrates a configuration of an information providing system 1 including an information providing apparatus 10 according to the fourth embodiment.

As illustrated in FIG. 9, the information providing system 1 includes the information providing apparatus 10 and a policy information database 20d. The policy information database 20d is a database that stores various data regarding various policies of a country, each local government, and the like.

The information providing apparatus 10 is an apparatus to which each device including the policy information database 20d is connected via a plug-in. Each device includes every device such as various servers, vehicles, and smartphones.

In the present embodiment, the information providing apparatus 10 performs learning of a model based on a data source acquired from the policy information database 20d. In the present embodiment, the information providing apparatus 10 performs a plug-in connection with the policy information database 20d (Step S1-3), and performs learning of a language model based on data stored in the policy information database 20d (Step S2-3).

In this way, the information providing apparatus 10 causes a language model to learn various data stored in the policy information database 20d. Thereby, the language model generates a response to an input prompt based on the learned data regarding policies. That is, the information providing apparatus 10 can generate a language model specialized in a policy field.

In the present embodiment, advice regarding a policy of a nation or each local government is given using a language model specialized in a policy field. Specifically, in the present embodiment, accounts (roles) of "Chairpersons A to C", "Experts A to C", and "Judge System" are set in advance by a system prompt of the language model.

"Chairpersons A to C" are accounts corresponding to heads of a nation or each local government. That is, Chairpersons A to C are accounts corresponding to a prime minister, a prefectural governor, a mayor, a town mayor, a village mayor, and the like. In the present embodiment, Chairperson A performs a discussion with a plurality of accounts such as Chairperson A-1, Chairperson A-2, and Chairperson A-3, and Chairperson B with Chairperson B-1, Chairperson B-2, and Chairperson B-3, and derives a policy plan as Chairperson A or Chairperson B.

"Experts A to C" are expert accounts of each field involved in operation of a nation or each local government.

Specific examples of an expert account include a financial field, a security field, an administrative field, an energy field, a healthcare field, and the like. "Judge System" is an account that judges a debate result by each account. For example, the judge system judges superiority or inferiority of an argument by each account, and proposes contents thereof. Expert Account A performs a debate with a plurality of accounts such as Expert Account A-1, Expert Account A-2, and Expert Account A-3, and Expert Account B with Expert Account B-1, Expert Account B-2, and Expert Account B-3, and derives a conclusion of a policy plan by each field.

In the present embodiment, an argument by each of a plurality of chairperson accounts is input to a language model as a prompt, and it is set as a system prompt so that other chairperson accounts debate against the input prompt.

By such setting, it becomes possible for each chairperson account to voluntarily perform a debate using a language model. As illustrated in FIG. 9, the information providing apparatus 10 performs a debate regarding politics by each account using a language model (Step S3-3).

For example, Chairperson Accounts A to C are accounts having different political philosophies such as conservative, reformist, right-wing, and left-wing, and each performs a debate using the language model. Virtual experts engage in billions of debates per second. This enables verification and problem solving from all angles, and allows proposal of policies etc. to be performed instantly.

Similarly, each expert account performs a debate with a plurality of accounts for each field regarding a field, and derives a conclusion regarding a policy plan as each field. Then, Chairperson Accounts A to C perform a debate regarding a policy plan based on debates of these respective fields. Note that the final debate may be performed by all accounts including each account.

For example, a chairperson account performs a debate regarding a policy, and "Judge System" performs a judge based on contents of the debate (Step S4-3). For example, "Judge System" is composed of a plurality of accounts. The plurality of accounts constituting a judge system perform a debate on debate contents of a chairperson account and the like, and judge a final policy. For example, "Judge System" determines the most appropriate policy plan by collating an argument by each account with data stored in the policy information database 20d when a predetermined termination condition is satisfied, such as when a debate boils down.

Then, "Judge System" proposes the determined policy plan to an actual chairperson. That is, in the information providing system 1, by using a language model, it is possible to simulate a huge amount of discussion regarding a policy.

In this way, the information providing apparatus 10 according to the embodiment generates a language model based on various findings registered in the policy information database 20d, performs a debate using a language model, and then makes a proposal regarding a policy plan.

Therefore, according to the information providing apparatus 10 according to the embodiment, it is possible to effectively utilize a language model specialized in a specific field.

Note that the above-described debate is performed by the following Steps 1 to 5.

### Step 1: Set multi-functional virtual experts

Virtual experts in different policy fields exist in hundreds of sets, and specific characters are set, for example, "Political Science Expert A", "Political Science Expert B", and "Political Science Expert C" for political science, and "Economics Expert A", "Economics Expert B", and "Economics Expert C" for economics.

### Step 2: Speed up information exchange

These experts perform billions of debates per second while exchanging information based on each data. Thereby, it is possible to instantly make judgments on optimal policies, economic measures, and the like.

### Step 3: Handling of data

The virtual experts proceed with a debate while sharing various types of information such as stock prices and birth rates. Therefore, an optimal solution from a multilateral perspective is quickly derived.

### Step 4: Self-learning and update

When new knowledge or information is discovered in a process of a debate, it is immediately reflected in the database and utilized for the debate from the next time. Thereby, diagnosis and treatment can be provided based on the latest policy information.

### Step 5: Formation of consensus

A debate forms a final "consensus" through collision of different opinions and perspectives. Thereby, when a policy proposal is performed, information and knowledge from various fields are comprehensively incorporated.

Although a chairperson account has been described above, respective characters are also set for the Expert A to C accounts, and each account is further divided into various characters, and each performs a debate, thereby forming a consensus as Experts A to C.

### (5. Fifth Embodiment)

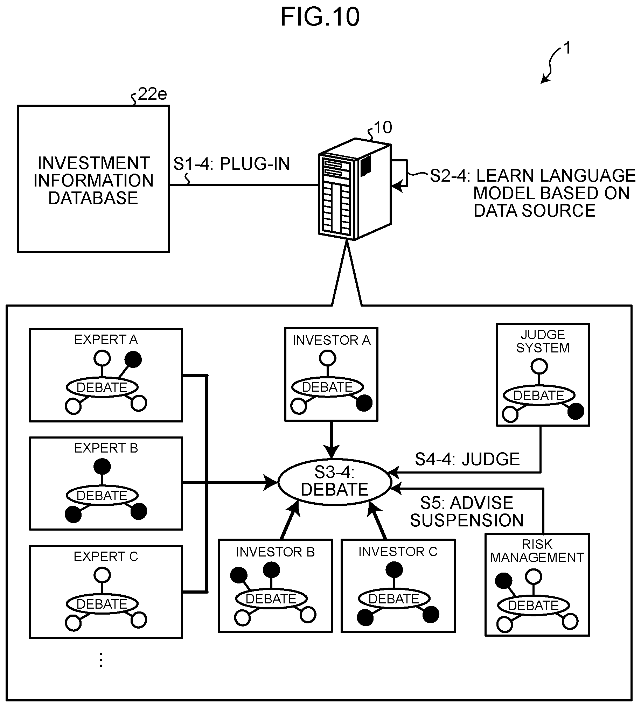

Next, a fifth embodiment will be described. FIG. 10 is a diagram explaining an outline of an information providing apparatus according to the embodiment. FIG. 10 illustrates a configuration of an information providing system 1 including the information providing apparatus 10 according to the embodiment.

As illustrated in FIG. 10, the information providing system 1 includes the information providing apparatus 10 and an investment information database 20e. The investment information database 20e is a database that stores various data regarding investment.

In the information providing system 1, the investment information database 20e stores information announced in each news media (Nikkei Stock Average, long-term and short-term interest rates, employment statistics, etc.) and information uniquely acquired by the information providing system 1.

The investment information database 20e uniquely acquires and stores, for example, information regarding ripeness of agricultural crops and the number of cars parked in parking lots of large supermarkets and the like from photo analysis of short-range satellites, POS data, individual warehouse information, credit card information, housing sales market data, and the like. In the present embodiment, the information providing apparatus 10 performs a plug-in connection with the investment information database 20e (Step S1-4), and performs learning of a language model based on data stored in the investment information database 20e (Step S2-4).

In this way, the information providing apparatus 10 causes a language model to learn various data stored in the investment information database 20e. Thereby, a language model generates a response to an input prompt based on the learned data regarding investment. That is, the information providing apparatus 10 can generate a language model specialized in an investment field.

In the present embodiment, advice regarding investment is given using a language model specialized in an investment field. Specifically, in the present embodiment, accounts (roles) of "Investors A to C", "Experts A to C", "Judge System", and "Risk Management" are set in advance by a system prompt of the language model.

Here, it is assumed that accounts in the present embodiment include bot accounts and human accounts. The bot account is a language model for which a system prompt can be set. The bot account outputs a response using a language model. On the other hand, a human account is a human. That is, a human account outputs a response according to an input of a human.

"Investors A to C", "Experts A to C", "Judge System", and "Risk Management" in FIG. 10 are upper accounts. The upper account includes at least one of a bot account or a human account. White circles in FIG. 10 represent bot accounts. Black circles in FIG. 10 represent human accounts.

Hereinafter, a bot account or a human account included in an upper account may be referred to as a lower account. In addition, when it is clear from the preceding and following descriptions and drawings, or when an upper account and a lower account are not distinguished, "upper" and "lower" are omitted and simply referred to as an account.

Hereinafter, description will be given assuming that all accounts are bot accounts. A configuration of FIG. 10 is realized by appropriately replacing a bot account with a human account or adding a human account as a lower account to an upper account.

"Investors A to C" are upper accounts corresponding to, for example, institutional investors or individual investors who operate so-called funds or the like. In the present embodiment, Investor A is composed of a plurality of lower accounts such as Investor A-1, Investor A-2, and Investor A-3, and Investor B is composed of Investor B-1, Investor B-2, and Investor B-3, etc., and Investor A derives a conclusion regarding an investment policy as Investor A by a debate by Investor A-1, Investor A-2, and Investor A-3. For example, Investor A-1 is a character that values inflation rate, Investor A-2 is a character that values employment statistics, and Investor A-3 is a character that values industrial production index, and each debates and derives a conclusion as Investor A. Thus, in the world, there are various indicators such as:
1. Inflation rate: Indicates the rate of increase in prices.
2. Unemployment rate: Percentage of unemployed people in the labor force.
3. GDP growth rate: Indicates growth of Gross Domestic Product.
4. Consumer Confidence Index: Consumers' confidence in the economy.
5. Industrial Production Index: Indicates production activities of manufacturing, mining, and utilities.
6. Number of employment increases: Number of newly employed people.
7. Real wages: Movement of wages considering price fluctuations.
8. Housing sales data: Sales status of new houses and existing houses.
9. Consumer Price Index (CPI): Indicates price fluctuations for general consumers.
10. Producer Price Index (PPI): Price fluctuations at the production stage.
11. Retail sales: Sales status of the retail industry.
12. Business inventories: Amount of inventory of goods and materials held by companies.
13. International trade balance: Difference between exports and imports.
14. Manufacturing order data: Order status of the manufacturing industry.
15. Non-manufacturing activity index: Indicates economic activities of service industries, etc.
16. Trends in financial markets: Stock prices, bond yields, currency prices, etc.
17. Trends in bank lending: Status of lending activities of banks.
18. Core inflation rate: Inflation rate excluding food and energy.
19. Wage growth rate: Indicates growth of average wages.
20. Trends in the world economy: Economic growth rates and policy trends of major countries.

It is a feature that investors are divided into those who value each of these indicators and perform a debate. "Experts A to C" are expert accounts of each field regarding investment.

Examples of each field regarding investment include a commodity field, a corporate bond field, a real estate field, an FX field, a portfolio field, and the like. "Judge System" is an account that judges a debate result by each account. For example, the judge system judges superiority or inferiority of an argument by each account, and proposes contents thereof.

In the present embodiment, an argument by each of a plurality of accounts is input to the language model as a prompt, and it is set as a system prompt so that other accounts debate against the input prompt.

By such setting, it becomes possible for each account to voluntarily perform a debate using a language model. The information providing apparatus 10 performs a debate regarding an investment policy by each account using a language model (Step S3-4).

For example, Investor Accounts A to C are accounts having different investment philosophies, and each performs a debate using a language model. Virtual experts engage in billions of debates per second. This enables verification and problem solving from all angles, and allows proposal of investment policies etc. to be performed instantly.

Similarly, each expert account performs a debate with a plurality of accounts for each field regarding the field, and derives a conclusion regarding an investment policy for each field. Then, an investor account performs a debate regarding an investment policy based on debates of these respective fields. Note that the final debate may be performed including all accounts including each account.

As a result of these debates, "Judge System" performs a judge based on contents of the debate (Step S4-4). For example, "Judge System" is composed of a plurality of accounts. The plurality of accounts constituting the judge system perform a debate on debate contents of the investor account and the like, and judge a final investment policy. For example, "Judge System" determines the most appropriate investment policy by collating an argument by each account with data stored in the investment information database 20e when a predetermined termination condition is satisfied, such as when a debate boils down.

Then, "Judge System" proposes a determined investment policy to an actual investor. That is, in the information providing system 1, by using a language model, it is possible to simulate a huge amount of discussion regarding an investment policy.

In addition, "Risk Management" performs a debate on risks in the current portfolio based on the latest information registered in the investment information database 20e. "Risk Management" is composed of a plurality of accounts, and a plurality of accounts constituting the risk management perform a debate on investment risks. As a result, "Risk Management" advises suspension of investment when a risk of investment exceeds a threshold (Step S5-4).

That is, the information providing apparatus 10 can avoid or minimize losses and the like in investment in advance by a function of "Risk Management".

In this way, the information providing apparatus 10 according to the embodiment generates a language model based on various findings registered in the investment information database 20e, performs a debate using a language model, and then makes a proposal regarding an investment policy.

Therefore, according to the information providing apparatus 10 according to the embodiment, it is possible to effectively utilize a language model specialized in a specific field.

Note that the above-described debate is realized by the following Steps 1 to 5.

### Step 1: Set multi-functional virtual experts

Virtual experts in different investment fields exist in hundreds of sets, and specific characters are set, for example, "Real Estate Expert A", "Real Estate Expert B", and "Real Estate Expert C" for the real estate field, and "FX Expert A", "FX Expert B", and "FX Expert C" for FX.

### Step 2: Speed up information exchange

These experts perform billions of debates per second while exchanging information based on each data. Thereby, it is possible to instantly make judgments on optimal investment policies and the like.

### Step 3: Handling of data

The virtual experts proceed with the debate while sharing various types of information such as stock prices. Therefore, an optimal solution from a multilateral perspective is quickly derived.

### Step 4: Self-learning and update

When new knowledge or information is discovered in the process of the debate, it is immediately reflected in the database and utilized for the debate from the next time. Thereby, an optimal investment policy can be provided based on the latest investment information.

### Step 5: Formation of consensus

The debate forms a final "consensus" through collision of different opinions and perspectives. Thereby, when an investment policy proposal is performed, information and knowledge from various fields are comprehensively incorporated.

Although the investor account has been described above, respective characters are also set for Expert A to C accounts, and each account is further divided into various characters, and each performs a debate, thereby forming a consensus as Experts A to C.

The information providing apparatus 10 judges a debate result by the information providing apparatus 10 by a judge account set in a language model, and proposes an investment policy. For example, the information providing apparatus 10 performs a judge when a predetermined termination condition is satisfied, such as when a debate by the information providing apparatus 10 boils down.

For example, the information providing apparatus 10 refers to various data registered in the investment information database 20e, judges an investment policy estimated to be most appropriate among investment policies debated by each investor account, and proposes the investment policy. Alternatively, the investment policy may be proposed by adding various consensuses such as a consensus as Expert A to the consensus result of the investor account.

In addition, when it is determined by a risk management account that the risk is high, the information providing apparatus 10 advises an investor to withdraw from investment or the like. Thereby, risks due to investment can be reduced.

### (Debate when human account is included)

Description has been given so far regarding a debate assuming that the lower account is a bot account. Here, a debate when a human account is included in the lower account will be described. In this case, processing contents of Step 1 and Step 2 described above by the information providing apparatus 10 change.

First, in Step 1, the information providing apparatus 10 changes a configuration so that at least some of the upper accounts include a human account as a lower account. For example, the information providing apparatus 10 replaces a bot account with a human account or newly adds a human account. A user, that is, an existing human is associated with the human account.

Subsequently, in Step 2, the information providing apparatus 10 presents a text output as a response by each account to a user associated with the human account. Then, the information providing apparatus 10 accepts an input of a text from the user. Note that a text of a response output by each account and an operation of outputting a text as a response are referred to as a "statement". The "statement" includes a text input by the user.

The bot account treats the statement of the human account in the same manner as a statement of a bot account. For example, the bot account inputs statements of other bot accounts and the human account to its own language model as a prompt, and further outputs a response.

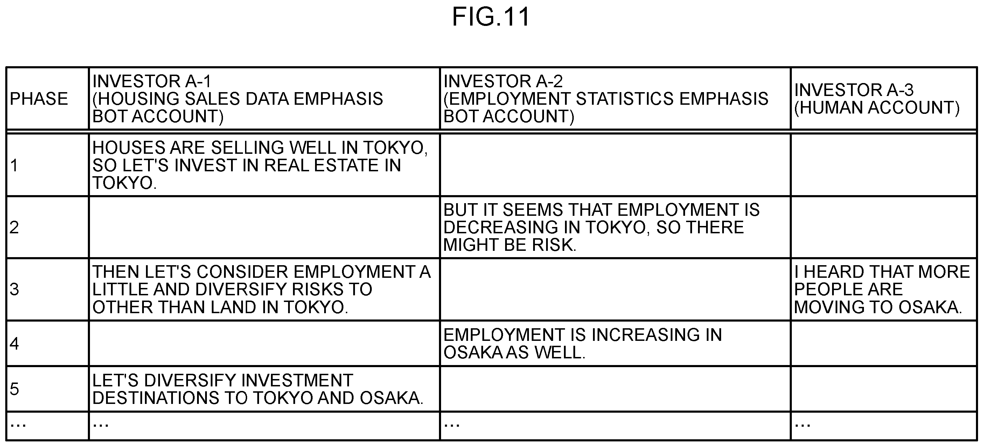

The processing of the information providing apparatus 10 will be described with reference to FIG. 11. FIG. 11 is a diagram explaining a debate. As illustrated in FIG. 11, the information providing apparatus 10 performs a debate according to progress of phases. Here, it is assumed that Investor A, which is an upper account, is composed of three lower accounts of Investor A-1, Investor A-2, and Investor A-3.

Investor A-1 is a bot account of a character that values housing sales data. For example, a language model of Investor A-1 values housing sales data as a basis and outputs a text regarding investment.

Investor A-2 is a bot account of a character that values employment statistics. For example, a language model of Investor A-2 values employment statistics as a basis and outputs a text regarding investment.

Note that the entity of the bot account is a language model, and a debate is realized by the information providing apparatus 10 repeating input of a prompt to the language model. The prompt is, for example, a statement of a language model in the previous phase. What kind of data is emphasized in making a statement may be set by a system prompt. As a result, each bot account has a different tendency of response.

Investor A-3 is a human account. A user is associated with Investor A-3. In addition, a character (what is emphasized) of Investor A-3 may be anything.

As illustrated in FIG. 11, in Phase 1, Investor A-1 states, "Houses are selling well in Tokyo, so let's invest in real estate in Tokyo.". Here, the information providing apparatus 10 advances a phase.

In Phase 2, Investor A-2 states, "But it seems that employment is decreasing in Tokyo, so there might be a risk." based on a result of inputting the statement of Investor A-1 in Phase 1 to the language model as a prompt.

Next, in Phase 3, a human account states, "I heard that the number of people living in Osaka is increasing.". The statement of the human account is contents input by a user. For example, the information providing apparatus 10 may display a statement of each account on a user's terminal by a chat UI, and accept an input of the user from an input field of the chat UI.

Note that each account including a bot account and a human account does not need to make a statement in all phases. The information providing apparatus 10 only needs to cause at least one account to make a statement in each phase.

In addition, when a human account is included in accounts performing a debate, it is difficult to cause a user to make a statement every time in each of phases that may number in the billions. Therefore, for example, the information providing apparatus 10 may accept a user's statement only in a specific phase, such as once every 10,000 phases.

In addition, the information providing apparatus 10 may accept a user's statement when a condition is satisfied. The condition is a case where the bot account repeats a statement of the same content a certain number of times, or the like. By a bot account using a user's statement as a prompt, it is expected that a stagnant debate starts to proceed toward a conclusion.

In this way, the information providing apparatus 10 presents a response to a user when a response satisfies a condition. Then, the information providing apparatus 10 collects a text input according to the presented response.

In addition, there is a case where a user has knowledge that was not used for learning of a language model of a bot account. In such a case, there is a possibility that a more useful conclusion can be obtained in a debate when a human account is included than in the case of only the bot account.

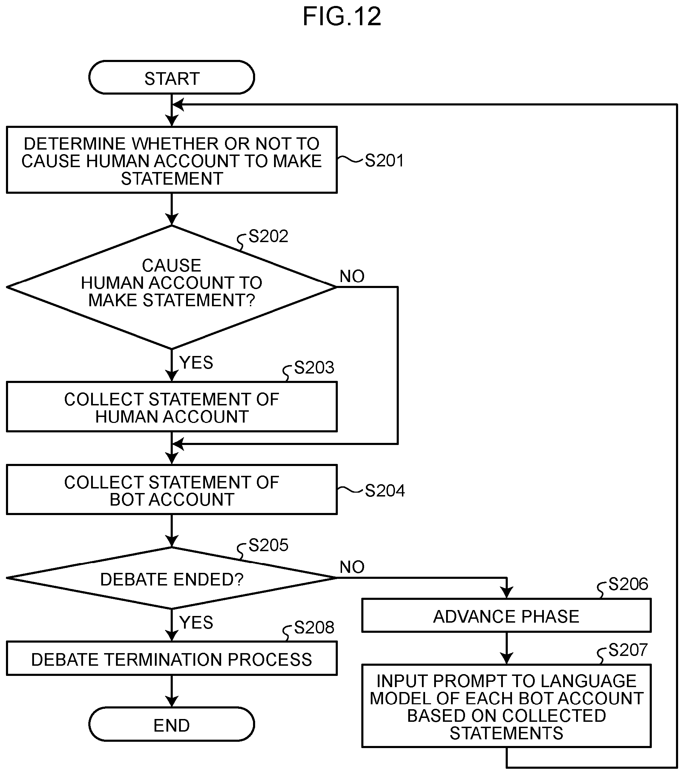

A processing procedure of the debate will be described with reference to FIG. 12. FIG. 12 is a flowchart illustrating a processing procedure of a debate. As illustrated in FIG. 12, when a phase starts, the information providing apparatus 10 determines whether or not to cause a human account to make a statement (Step S201). Conditions for causing a human account to make a statement are that a prescribed number of phases have elapsed, that a bot account repeats the same statement a certain number of times, and the like.

When not causing a human account to make a statement (Step S202, No), the information providing apparatus 10 proceeds to Step S204. On the other hand, when causing a human account to make a statement (Step S202, Yes), the information providing apparatus 10 collects a statement of the human account (Step S203). For example, the information providing apparatus 10 collects a text input by a user in an input field of a chat UI as a statement of the human account. In addition, the information providing apparatus 10 may present a statement of each account to the user immediately before Step S203.

Subsequently, the information providing apparatus 10 collects a statement of a bot account (Step S204). Here, when a debate has ended (Step S205, Yes), the information providing apparatus 10 performs a debate termination process (Step S208). For example, the information providing apparatus 10 may determine that the debate has ended when a prescribed number of phases have elapsed or when a judge by the judge account is performed. The debate termination process is to shift to a debate between upper accounts (for example, Step S3 in FIG. 1).

When the debate has not ended (Step S205, No), the information providing apparatus 10 advances a phase (Step S206). Then, it inputs a prompt to a language model of each bot account based on the collected statements (Step S207), returns to Step S201, and performs processing in the phase after progress.

### (6. Sixth Embodiment)

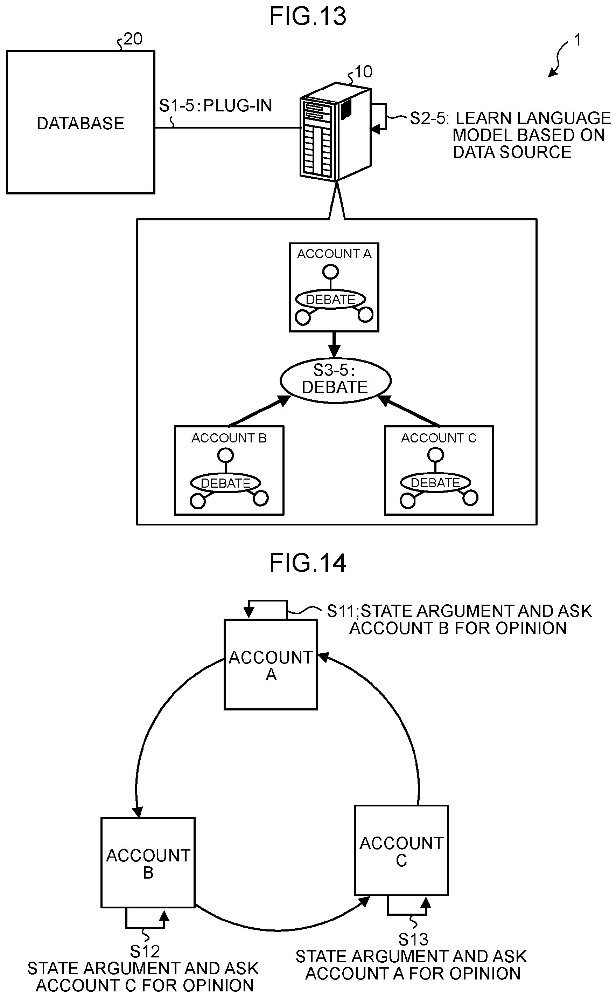

Next, a sixth embodiment will be described. FIG. 13 is a diagram explaining an outline of an information providing apparatus according to the sixth embodiment.

As illustrated in FIG. 13, the information providing system 1 includes the information providing apparatus 10 and a database 20. The database 20 is a database that stores various data. Note that the database 20 may be composed of a plurality of databases, or may be configured inside the information providing apparatus 10.

The information providing apparatus 10 is an apparatus to which each device including the database 20 is connected via a plug-in. Each device includes every device such as various servers, vehicles, and smartphones. That is, data is transmitted to the database 20 in real time from every device such as various servers, vehicles, and smartphones, and the database 20 stores these data.

In the present embodiment, the information providing apparatus 10 performs a plug-in connection with the database 20 (Step S1-5), and performs learning of a language model based on data stored in the database 20 (Step S2-5).

As described above, since data stored in the database 20 is constantly updated, a language model learned using various data stored in the database 20 can generate a response based on the latest data.

In the present embodiment, a proposal is made regarding a debate using a language model. Specifically, as illustrated in FIG. 13, the information providing apparatus 10 sets a plurality of accounts in a language model by a prompt input to the language model, and then performs a debate by the plurality of accounts (Step S3-5).

In the example illustrated in FIG. 13, a scene where a total of three accounts of Accounts A to C are set is shown. In addition, for example, as illustrated in FIG. 13, Accounts A to C may be composed of a plurality of accounts respectively, and a conclusion as Accounts A to C may be derived after performing a debate by each account.

Next, an outline of a debate by each account will be described with reference to FIG. 14. FIG. 14 is a diagram illustrating an outline of a debate according to the embodiment. In the present embodiment, a method for performing a debate by each account using a language model more efficiently is proposed.

Specifically, as illustrated in FIG. 14, first, Account A states an argument as Account A regarding a specific agenda and asks Account B for an opinion (Step S11). Account B, who was asked for an opinion by Account A, states an argument as Account B and asks Account C for an opinion (Step S12).

At this time, Account B agrees with a part of Account A's argument that can be agreed upon after stating the reason, and states an argument as Account B from a viewpoint different from that of Account A.

Account C, who was asked for an opinion by Account B, states an argument as Account C and then asks Account A for an opinion (Step S13). At this time, Account C agrees with parts of Account A's and Account B's arguments that can be agreed upon after stating the reason, and states an argument as Account C from a viewpoint different from those of Account A and Account B.

For example, Account C theoretically agrees with points where it can agree with either Account A or Account B or both, and states an argument as Account C if there is an argument from a completely different viewpoint from another angle of an agreeable range.

In addition, when there is no argument from a different viewpoint, Account C clarifies which opinion of Account A or Account B it agrees with.

Thereafter, Account A states an argument against arguments of Account B and Account C, and then asks Account B for an opinion. At this time, Account A agrees with parts of Account B's and Account C's arguments that can be agreed upon, gives further logical explanation for the initial argument of Account A, or states an argument as Account A from a viewpoint different from those of Account B and Account C.

By such a loop of debate, Accounts A to C make logically explainable arguments from different viewpoints respectively, and agree with arguments of other accounts when there are no more logically explainable arguments.

Then, finally, each account agrees with an argument by any of the accounts. In the present embodiment, a state where all accounts agree is referred to as a state where a consensus is obtained. Then, the information providing apparatus 10 provides information regarding such a debate (sentences regarding responses of each account, etc.) to a user or the like. For example, a user can avoid conflict due to an argument different from others by making a decision based on information regarding such a debate.

In this way, in the information providing apparatus 10 according to the embodiment, each account states opinions multilaterally regarding the agenda, and performs a debate until a consensus is finally obtained.

Therefore, since the information providing apparatus 10 according to the embodiment can perform a simulation regarding a debate for obtaining a consensus in an arbitrary group having different opinions, for example, it is possible to effectively utilize a language model.

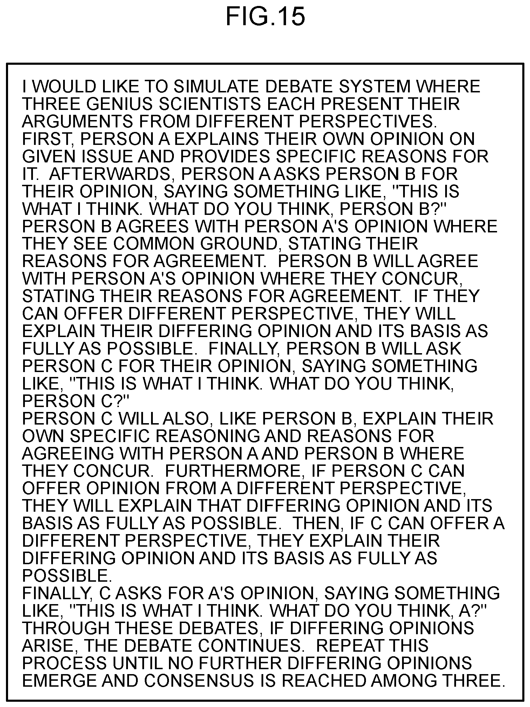

Next, an example of a prompt input to a language model will be described with reference to FIG. 15. FIG. 15 is a diagram illustrating an example of a prompt according to the sixth embodiment. As illustrated in FIG. 15, the prompt is text information regarding preconditions for a debate by a plurality of accounts (here, three genius scientists).

As illustrated in FIG. 15, for example, Mr. A, who is a genius scientist, states an opinion and then asks Mr. B for an opinion, saying "I think this way. What do you think, Mr. B?", and Mr. B, who is a genius scientist, also asks Mr. C for an opinion, saying "I think this way. What do you think, Mr. C?", similarly to Mr. A.

Similarly to Mr. A and Mr. B, Mr. C also states an opinion and then asks Mr. A for an opinion, saying "I think this way. What do you think, Mr. A?". At this time, Mr. A to Mr. C respectively agree with opinions of others or state opinions different from others.

Through these debates, finally, a debate is performed until no different opinions appear, that is, until all accounts agree with any one of opinions of Mr. A to Mr. C, and a consensus is obtained.

By such a prompt, each account set in the language model makes its own argument and performs a debate until a consensus is obtained. Note that, although the example of FIG. 3 shows a case where the plurality of accounts are "genius scientists", it is not limited to this. That is, arbitrary accounts may be set. In this case, the arbitrary accounts may be accounts of the same attribute or accounts of different attributes. Specifically, for example, assuming a case of debating a medical policy for a patient, each account may be a doctor, a patient, a family, etc., and in this case, the doctor may be further subdivided into surgery, internal medicine, dermatology, etc.

The information providing apparatus 10 repeats a debate until a consensus by a plurality of accounts is obtained after the plurality of accounts set in a language model that generates a response to an input prompt respectively assert opinions and then ask other accounts for opinions.

For example, the information providing apparatus 10 sets a plurality of accounts in a language model by inputting a prompt as illustrated in FIG. 15 to the language model, and starts a debate by the plurality of accounts. At this time, a plurality of accounts may be set by a user or may be set by the language model. In addition, an agenda of a debate may be set by a user or may be set by the language model.

Note that, for example, when causing a language model to set a plurality of accounts, a debate is performed by, for example, the following Step 1 to Step 5. In the following, a case where a plurality of doctor accounts debate a medical policy for an actually existing patient as an agenda will be described.

### Step 1: Set multi-functional virtual experts

Virtual experts exist in hundreds of sets, and specific characters are set, for example, "Neurology Expert A", "Neurology Expert B", and "Neurology Expert C" for neurology, and "Cardiology Expert A", "Cardiology Expert B", and "Cardiology Expert C" for cardiology.

### Step 2: Speed up information exchange

These experts perform billions of debates per second while exchanging information based on patient data. Thereby, it is possible to instantly make judgments on a patient's condition, symptoms, treatment methods, and the like.

### Step 3: Handling of data

The virtual experts proceed with a debate while sharing various types of information such as MRI data, genetic data, and blood data. Therefore, an optimal solution from a multilateral perspective is quickly derived.

### Step 4: Self-learning and update

When new knowledge, information, or a treatment method is discovered in the process of the debate, it is immediately reflected in the database 20 and utilized for the debate from the next time. Thereby, diagnosis and treatment can be provided based on the latest medical information.

### Step 5: Formation of consensus

The debate forms a final "consensus" through collision of different opinions and perspectives. Thereby, when diagnosis or treatment proposal to the patient is performed, information and knowledge from various fields are comprehensively incorporated. There may be a plurality of treatment proposal policies.

Through these debates, for example, a consensus regarding a medical policy for a patient by a plurality of doctor accounts is obtained. Note that, for example, a plurality of accounts involved in medical care of the patient, including a patient account and a family account, may be allowed to participate in the debate.

The information providing apparatus 10 provides information regarding the above-described debate to a user or the like. For example, the information providing apparatus 10 provides a response output by a language model as each account as information regarding a debate.

Thereby, the user can confirm contents of the debate and make a decision based on these contents.

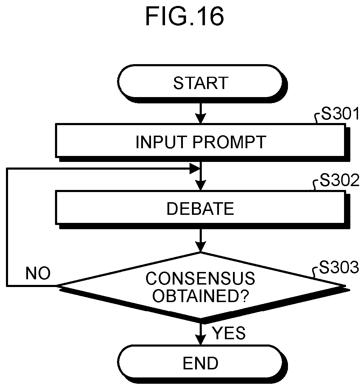

Next, a processing procedure executed by the information providing apparatus 10 according to the sixth embodiment will be described with reference to FIG. 16. FIG. 16 is a flowchart illustrating a processing procedure executed by the information providing apparatus 10 according to the sixth embodiment.

As illustrated in FIG. 16, the information providing apparatus 10 first inputs a prompt to the language model (Step S301). Subsequently, the information providing apparatus 10 performs a debate using the language model (Step S302).

Subsequently, the information providing apparatus 10 determines whether or not a consensus has been obtained (Step S303). When determining that a consensus has been obtained (Step S303; Yes), the information providing apparatus 10 ends the process, and when determining that a consensus has not been obtained (Step S303; No), returns to the process of Step S302 and repeats a debate.

According to the present embodiment, since a debate is performed until a consensus is obtained using a language model learned based on data stored in a database, it is possible to effectively utilize a language model.

Although the present invention has been described above using the embodiments, the technical scope of the present invention is not limited to the scope described in the above embodiments. It is apparent to those skilled in the art that various modifications or improvements can be added to the above embodiments. It is apparent from the description of the claims that forms to which such modifications or improvements are added can also be included in the technical scope of the present invention.

It should be noted that the execution order of each process such as operations, procedures, steps, and stages in the apparatus, system, program, and method shown in the claims, specification, and drawings can be realized in any order unless particularly explicitly indicated as "before", "prior to", or the like, and unless an output of a previous process is used in a subsequent process. Even if the operation flow in the claims, specification, and drawings is described using "first,", "next,", or the like for convenience, it does not mean that it is essential to implement in this order.

### List of Reference Signs

1 Information providing system
10 Information providing apparatus
11 Communication unit
12 Storage unit
13 Control unit
20 Database
20a Legal case database
20b Accounting case database
20c Medical case database
20d Policy information database
20e Investment information database
31 Acquisition unit
32 Generation unit
33 Debate unit
34 Judging unit
121 Language model information

## Claims

1. An information providing apparatus comprising:
a debate unit that performs a debate regarding a legal case by a plurality of lawyer accounts that make respectively different arguments, the lawyer accounts being set in a language model that is generated by learning data stored in a database regarding legal cases and that generates a response to an input prompt; and
a judging unit that judges superiority or inferiority of the arguments based on a result of the debate by the debate unit, by a judging account set in the language model.

2. The information providing apparatus according to claim 1, wherein
the debate unit performs the debate including a public prosecutor account set in the language model.

3. The information providing apparatus according to claim 1, wherein
the judging unit judges with reference to related laws or past judicial precedents stored in the database.

4. The information providing apparatus according to claim 1, wherein
the debate unit debates necessity of a lawsuit by a client company.

5. The information providing apparatus according to claim 1, wherein
the debate unit debates contents of a lawsuit assumed between a client company and another company, and contract contents for avoiding the lawsuit or winning the lawsuit, and
the judging unit creates a draft of the contract contents based on a judge result.

6. An information providing method executed by an information providing apparatus, the method comprising:
a debate step of performing a debate regarding a legal case by a plurality of lawyer accounts that make respectively different arguments, the lawyer accounts being set in a language model that is generated by learning data stored in a database regarding legal cases and that generates a response to an input prompt; and
a judging step of judging superiority or inferiority of the arguments based on a result of the debate by the debate step, by a judging account set in the language model.

7. An information providing program for causing a computer to execute:
a debate step of performing a debate regarding a legal case by a plurality of lawyer accounts that make respectively different arguments, the lawyer accounts being set in a language model that is generated by learning data stored in a database regarding legal cases and that generates a response to an input prompt; and
a judging step of judging superiority or inferiority of the arguments based on a result of the debate by the debate step, by a judging account set in the language model.

8. An information providing apparatus comprising:
a debate unit that performs a debate regarding accounting by a plurality of accountant accounts that make different arguments, the accountant accounts being set in a language model that is generated by learning data stored in a database regarding accounting cases and that generates a response to an input prompt; and
a judging unit that judges superiority or inferiority of the arguments based on a result of the debate by the debate unit, by a judging account set in the language model.

9. An information providing apparatus comprising:
a debate unit that performs a debate regarding a treatment policy for a patient by a plurality of doctor accounts set in a language model that is generated by learning data stored in a database regarding medical care and that generates a response to an input prompt; and
a proposal unit that judges a debate result by the debate unit by a judge account set in the language model, and proposes the treatment policy.

10. An information providing apparatus comprising:
a debate unit that performs a debate regarding a treatment policy for a patient by a plurality of doctor accounts set in a language model that is generated by learning data stored in a database regarding medical care and that generates a response to an input prompt; and
a proposal unit that judges a debate result by the debate unit by a judge account set in the language model, and proposes the treatment policy, wherein
the proposal unit generates minutes of the debate that led to a result of the treatment policy, and provides the minutes together with the treatment policy.

11. An information providing apparatus comprising:
a debate unit that performs a debate regarding a treatment policy for a patient by a plurality of doctor accounts set in a language model that is generated by learning data stored in a database regarding medical care and that generates a response to an input prompt; and
a proposal unit that judges a debate result by the debate unit by a judge account set in the language model, and proposes the treatment policy, wherein
the debate unit restricts access to the data by the language model in the debate.

12. An information providing apparatus comprising:
a collection unit that collects responses output by a plurality of language models that output responses regarding a treatment policy for a patient based on an input prompt and are set so that tendencies of the responses are mutually different, and a text input by a user;
a presentation unit that presents the collected responses to the user; and
an output control unit that inputs the collected responses and the text to the plurality of language models as a prompt, and causes the plurality of language models to further output responses.

13. An information providing apparatus comprising:
a debate unit that performs a debate regarding a management policy of a company by a plurality of executive officer accounts set in a language model that is generated by learning data stored in a database regarding corporate activities and that generates a response to an input prompt; and
a proposal unit that judges a debate result by the debate unit by a judge account set in the language model, and proposes the management policy.

14. An information providing apparatus comprising:
a debate unit that performs a debate regarding a policy plan by a plurality of chairperson accounts set in a language model that is generated by learning data stored in a database regarding politics and that generates a response to an input prompt; and
a proposal unit that judges a debate result by the debate unit by a judge account set in the language model, and proposes the policy plan.

15. An information providing apparatus comprising:
a debate unit that performs a debate regarding an investment policy by a plurality of investor accounts set in a language model that is generated by learning data stored in a database regarding investment and that generates a response to an input prompt; and
a proposal unit that judges a debate result by the debate unit by a judge account set in the language model, and proposes the investment policy.

16. An information providing apparatus comprising:
a debate unit that performs a debate regarding an investment policy by a plurality of investor accounts set in a language model that is generated by learning data stored in a database regarding investment and that generates a response to an input prompt; and
a proposal unit that judges a debate result by the debate unit by a judge account set in the language model, and proposes the investment policy, wherein
the proposal unit generates minutes of the debate that led to a result of the investment policy, and provides the minutes together with the investment policy.

17. An information providing apparatus comprising:
a debate unit that performs a debate regarding an investment policy by a plurality of investor accounts set in a language model that is generated by learning data stored in a database regarding investment and that generates a response to an input prompt; and
a proposal unit that judges a debate result by the debate unit by a judge account set in the language model, and proposes the investment policy, wherein
the debate unit restricts access to the data by the language model in the debate.

18. An information providing apparatus comprising:
a debate unit that repeats a debate until a consensus by a plurality of accounts is obtained after the plurality of accounts set in a language model that generates a response to an input prompt respectively assert opinions and then ask other accounts for opinions; and
a providing unit that provides information regarding contents of the debate by the debate unit.
